# EUROPEAN PATENT APPLICATION

(11) **EP 4 778 529 A1**
(43) Date of publication of application: **22.07.2026**
(21) Application number: 24865567.2
(22) Date of filing: 13.09.2024
(51) Int. Cl.: A61K 31/7088, A61K 35/76, A61K 35/761, A61K 38/45, A61K 48/00, A61P 9/00, A61P 9/10, C12N 7/01, C12N 15/12, C12N 15/864

(54) **PHARMACEUTICAL COMPOSITION FOR MYOCARDIAL REGENERATION, SUPPRESSION OF MYOCARDIAL FIBROSIS, OR TREATMENT OF HEART DISEASE**

(30) Priority: 14.09.2023 JP 2023149419
(71) Applicant: Kyoto University, Kyoto-shi, Kyoto 606-8501 (JP)
(72) Inventor: ASHIDA, Noboru, Kyoto-shi, Kyoto 606-8501 (JP)
(74) Representative: Hoffmann Eitle
(86) International application number: PCT/JP2024/032924
(87) International publication number: WO 2025/058068

(57) **Abstract**

The present disclosure provides a pharmaceutical composition for regenerating myocardium, suppressing myocardial fibrosis, or treating a cardiac disease comprising a polynucleotide encoding a constitutively active IKKβ.

## Description

### TECHNICAL FIELD

This patent application claims the priority of Japanese Patent Application No. 2023-149419, the entire disclosure of which is incorporated herein by reference.

The present disclosure relates to a pharmaceutical composition for regenerating myocardium, suppressing myocardial fibrosis, or treating a cardiac disease.

### BACKGROUND ART

In cardiac diseases such as myocardial infarction and dilated cardiomyopathy, cardiomyocytes responsible for pump function are damaged and eventually undergo degeneration or necrosis, resulting in myocardial remodeling due to fibrosis of the myocardial tissue. It is well known that these changes often lead to heart failure and arrhythmia, and consequent cardiac death. Cardiomyocytes generally lack a self-regenerative ability, and myocardial regeneration in mammals is observed only during the early postnatal period (Non-Patent Documents 1 and 2).

NF-κB is a transcription factor involved in inflammation, cell proliferation, and apoptosis. Activation of NF-κB is controlled by the inhibitory subunit, IκB, and requires phosphorylation, ubiquitination, and degradation of IκB. An IκB kinase (IKK) complex responsible for the initiation stage of NF-κB activation consists of three subunits: IKKα, IKKβ (also referred to as IKK-2), and IKKγ. A myofibroblast-selective IKKβ knockout mouse is known as a scleroderma model (Patent Document 1). A mutant of IKKβ that exhibits a constitutive kinase activity is known (Non-Patent Document 3).

### PRIOR ART DOCUMENT

### PATENT DOCUMENT

Patent Document 1: WO 2014/069597

### NON-PATENT DOCUMENT

Non-Patent Document 1: Qi Xiao, et al., A p53-based genetic tracing system to follow postnatal cardiomyocyte expansion in heart regeneration, Development (2017) 144, 580-589
Non-Patent Document 2: Vanessa Hertig, et al., p38α MAPK inhibition translates to cell cycle re-entry of neonatal rat ventricular cardiomyocytes and de novo nestin expression in response to thrombin and after apex resection, Scientific Reports (2019) 9:8203
Non-Patent Document 3: Mercurio F, et al. IKK-1 and IKK-2: cytokine-activated IkappaB kinases essential for NF-kappaB activation. Science. 1997 Oct 31;278(5339):860-6.

### SUMMARY OF THE INVENTION

### PROBLEMS TO BE SOLVED BY THE INVENTION

An object of the present disclosure is to provide a pharmaceutical composition for regenerating myocardium, suppressing myocardial fibrosis, or treating a cardiac disease.

### MEANS FOR SOLVING THE PROBLEMS

The present inventor has found that IKKβ that exhibits a constitutive kinase activity (constitutively active IKKβ) leads to suppression of myocardial fibrosis and myocardial regeneration, and is effective for treating cardiac diseases.

Accordingly, in one aspect, the present disclosure provides a pharmaceutical composition for treating a cardiac disease comprising a polynucleotide encoding a constitutively active IKKβ.

In one aspect, the present disclosure provides a pharmaceutical composition for regenerating myocardium comprising a polynucleotide encoding a constitutively active IKKβ.

In one aspect, the present disclosure provides a pharmaceutical composition for suppressing myocardial fibrosis comprising a polynucleotide encoding a constitutively active IKKβ.

### EFFECTS OF THE INVENTION

The present disclosure provides a new means for regenerating myocardium, suppressing myocardial fibrosis, or treating a cardiac disease.

### BRIEF DESCRIPTION OF THE DRAWINGS

[Fig. 1] Fig. 1 shows a method for producing CAIKKβ mice in which myofibroblasts express a constitutively active IKKβ.
[Fig. 2] Fig. 2 shows representative images of Masson's trichrome staining of heart sections 14 days after myocardial infarction surgery in control mice and CAIKKβ mice.
[Fig. 3] Fig. 3 shows representative images of immunostaining with a troponin T antibody of heart sections 14 days after myocardial infarction surgery in control mice and CAIKKβ mice.

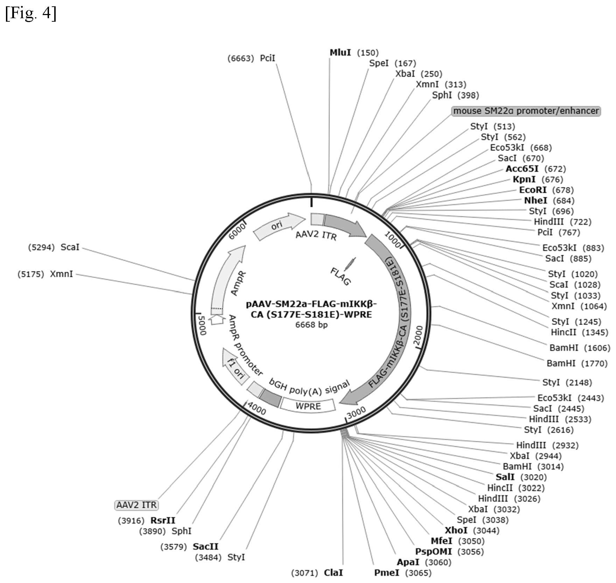

[Fig. 4] Fig. 4 shows a vector map of pAAV-SM22α-mIKKβ-CA-WPRE.
[Fig. 5] Fig. 5 shows echocardiographic images and changes over time of left ventricular ejection fraction in mice treated with SM22α-mTKKβCA-AAV9 two days after myocardial infarction surgery.
[Fig. 6] Fig. 6 shows survival rates of mice treated with a high dose or a low dose of SM22α-mIKKβCA-AAV9 two days after myocardial infarction surgery and control mice. Logrank test: among three groups p=0.000168, treatment vs no treatment p=0.0000658, dose 2×10¹¹ vg vs 5×10¹¹: p=0.0499.
[Fig. 7] Fig. 7 shows a representative image of Masson's trichrome staining of heart sections 142 days after myocardial infarction surgery in a mouse treated with SM22α-mIKKβCA-AAV9 two days after the surgery.
[Fig. 8] Fig. 8 shows representative images of Masson's trichrome staining of heart sections 176 days after myocardial infarction surgery in a mouse treated with SM22α-mIKKβCA-AAV9 two days after the surgery.
[Fig. 9] Fig. 9 shows representative images of Masson's trichrome staining of heart sections 142 days after myocardial infarction surgery in a mouse treated with SM22α-mIKKβCA-AAV9 two days after the surgery.
[Fig. 10] Fig. 10 shows a representative image of Masson's trichrome staining of heart sections 14 days after myocardial infarction surgery in a mouse (left), and a representative image of Masson's trichrome staining of heart sections 142 days after myocardial infarction surgery in a mouse treated with SM22α-mIKKβCA-AAV9 two days after the surgery (right).
[Fig. 11] Fig. 11 shows enlarged views of the boxed regions in Fig. 10.

### DETAILED DESCRIPTION

Unless otherwise defined, the terms used herein are read as generally understood by those skilled in the fields such as organic chemistry, medical sciences, pharmaceutical sciences, molecular biology, and microbiology. Definitions of several terms used herein are provided below, and these definitions shall prevail over the general understanding herein.

When a numerical value is preceded by the term "about", the value is intended to represent any value in the range of -10% of the value to +10% of the value. For example, "about 20" means "a value from 18 to 22." A numerical range includes all values between the two endpoints and the values of the endpoints. The term "about," when used in connection with a range, applies to both endpoints of the range. For example, "about 20 to 30" includes "18 to 33."

IKKβ is one of the subunits constituting the IκB kinase complex that activates NF-κB. The IκB kinase complex includes IKKα, IKKβ, and IKKγ, and stimulation by various cytokines generates the kinase activity of the IκB kinase complex. Amino acid sequences of human and mouse IKKβ are registered, for example, as GenBank accession numbers NP_001547.1 (SEQ ID NO: 1) and NP_001153246.1 (SEQ ID NO: 2), respectively. In the present disclosure, IKKβ includes products of alleles occurring naturally.

In the present disclosure, "constitutively active IKKβ" means IKKβ that exhibits a kinase activity in the absence of cytokine stimulation. For example, the constitutively active IKKβ exhibits a kinase activity of at least about 10% as compared with the kinase activity of IKKβ stimulated by cytokines, for example, about 20% or more, about 30% or more, about 40% or more, about 50% or more, about 60% or more, about 70% or more, about 80% or more, about 90% or more, about 95% or more, about 97%, about 98%, about 99%, or about 100% or more. A kinase activity can be measured by a known kinase assay, for example, the method described in Non-Patent Document 3.

Examples of the constitutively active IKKβ include a mutant IKKβ (SEQ ID NO: 3), which corresponds to SEQ ID NO: 1 in which serines at positions 177 and 181 of SEQ ID NO: 1 are substituted with glutamic acids, and a mutant IKKβ (SEQ ID NO: 4), which corresponds to SEQ ID NO: 2 in which serines at positions 177 and 181 of SEQ ID NO: 2 are substituted with glutamic acids. In one embodiment, the constitutively active IKKβ comprises, or consists of, an amino acid sequence that has about 60% or more identity, preferably about 70% or more identity, more preferably about 80% or more identity, still more preferably about 90% or more identity, particularly preferably about 95% or more identity, and most preferably about 97%, about 98%, or about 99% or more identity, to the amino acid sequence of SEQ ID NO: 3 or 4 when calculated using BLAST or the like (for example, using the default parameters, i.e., the parameters of the initial conditions, of BLAST), and in which the amino acids corresponding to positions 177 and 181 of SEQ ID NO: 3 or 4 are glutamic acids.

In one embodiment, the constitutively active IKKβ may comprise a sequence in which one or several amino acids are deleted, substituted, or added in the amino acid sequence of SEQ ID NO: 3 or 4, as long as the amino acids corresponding to positions 177 and 181 of SEQ ID NO: 3 or 4 are glutamic acids. "Several" amino acids mean preferably 2 to 7, more preferably 2 to 5, and most preferably 2 to 3 amino acids. An amino acid substitution is preferably a conservative substitution between similar amino acid residues.

An amino acid position in a second amino acid sequence "corresponding to" an amino acid position in a first amino acid sequence means a position in the second amino acid sequence that matches the position in the first amino acid sequence when the first and second amino acid sequences are aligned so as to maximize amino acid matches.

In one embodiment, the constitutively active IKKβ comprises the amino acid sequence of SEQ ID NO: 3 or 4. In one embodiment, the constitutively active IKKβ consists of the amino acid sequence of SEQ ID NO: 3 or 4.

In one embodiment, the polynucleotide encoding the constitutively active IKKβ comprises, or consists of, the nucleotide sequence of SEQ ID NO: 5, which encodes the amino acid sequence of SEQ ID NO: 3. In one embodiment, the polynucleotide encoding the constitutively active IKKβ comprises, or consists of, the nucleotide sequence of SEQ ID NO: 6, which encodes the amino acid sequence of SEQ ID NO: 4.

The polynucleotide encoding the constitutively active IKKβ may comprise, or consist of, a nucleotide sequence having about 60% or more identity, preferably about 70% or more identity, more preferably about 80% or more identity, still more preferably about 90% or more identity, particularly preferably about 95% or more identity, and most preferably about 97%, about 98%, or about 99% or more identity, to the nucleotide sequence of SEQ ID NO: 5 or 6 when calculated using BLAST or the like (for example, using the default parameters, i.e., the parameters of the initial conditions, of BLAST), and encoding the constitutively active IKKβ in which the amino acids corresponding to positions 177 and 181 of SEQ ID NO: 3 or 4 are glutamic acids.

The polynucleotide encoding the constitutively active IKKβ may have a nucleotide sequence that hybridizes, under stringent conditions, to a polynucleotide having a nucleotide sequence complementary to the nucleotide sequence of SEQ ID NO: 5 or 6, and encodes the constitutively active IKKβ in which the amino acids corresponding to positions 177 and 181 of SEQ ID NO: 3 or 4 are glutamic acids. Regarding "hybridize under stringent conditions", the hybridization can be carried out according to any conventional method, for example, those described in Molecular Cloning, T. Maniatis et al., CSH Laboratory (1983). The stringent conditions may be conditions where the hybridization is conducted in a solution containing 6 x SSC and 50% formamide at 45°C, wherein 10 x SSC is a solution containing 1.5 M NaCl and 0.15 M trisodium citrate, followed by washing in 2 x SSC at 50°C (Molecular Biology, John Wiley & Sons, N. Y. (1989), 6.3.1-6.3.6) or conditions that provides an equivalent stringency.

The polynucleotide encoding the constitutively active IKKβ may be operably linked to a promoter. In the present disclosure, "operably linked" means that a regulatory sequence element such as a promoter and the polynucleotide encoding the constitutively active IKKβ are linked in a manner that enables expression of the constitutively active IKKβ; they may be directly linked, or they may have any nucleotide sequence between them.

The promoter may be a constitutive promoter, an inducible promoter, or a tissue- or cell-specific promoter. Examples of constitutive promoters include a CMV (cytomegalovirus) promoter, a PGK (phosphoglycerate kinase) promoter, an EF1α (elongation factor 1-alpha) promoter, a CAG promoter, an SV40 promoter, and an MSCV promoter. In one embodiment, the polynucleotide encoding the constitutively active IKKβ is operably linked to a myofibroblast-specific promoter. Examples of myofibroblast-specific promoters include promoters of SM22α, collagen, αSMA, desmin, osteopontin, FSP-1, and HSP47.

Examples of human and mouse SM22α promoters include promoters consisting of the nucleotide sequences of SEQ ID NO: 7 and 8, respectively. In one embodiment, the SM22α promoter may comprise a nucleotide sequence having about 60% or more identity, preferably about 70% or more identity, more preferably about 80% or more identity, still more preferably about 90% or more identity, particularly preferably about 95% or more identity, and most preferably about 97%, about 98%, or about 99% or more identity, to the nucleotide sequence of SEQ ID NO: 7 or 8. In one embodiment, the SM22α promoter comprises the nucleotide sequence of SEQ ID NO: 7 or 8. In one embodiment, the SM22α promoter consists of the nucleotide sequence of SEQ ID NO: 7 or 8.

The polynucleotide encoding the constitutively active IKKβ may be DNA or RNA, and may comprise one or more non-natural nucleotides.

In one embodiment, the polynucleotide encoding the constitutively active IKKβ is RNA. In this embodiment, the polynucleotide may include a 5' cap structure, a 5' untranslated region (UTR), a sequence encoding the constitutively active IKKβ, a 3' UTR, and a poly(A) sequence. Such a polynucleotide can be produced, for example, by in vitro transcription using plasmid DNA as a template. For delivery into cells, the polynucleotide may be administered encapsulated in lipid particles or polymer particles.

The polynucleotide encoding the constitutively active IKKβ may be comprised in a vector. The vector can deliver a polynucleotide of a desired sequence into cells, and may be a viral vector or a non-viral vector. Examples of viral vectors include retroviral vectors, lentiviral vectors, adenoviral vectors, adeno-associated virus (AAV) vectors, herpes simplex virus (HSV) vectors, vaccinia virus vectors, Sendai virus vectors, and measles virus vectors. Examples of non-viral vectors include plasmid vectors, liposome vectors, and artificial chromosomes (e.g., YAC, BAC, and PAC). Each vector can be prepared according to a conventional method.

The vector may comprise one or more regulatory sequences that regulate expression of the constitutively active IKKβ. Examples of regulatory sequences include promoters, enhancers, polyadenylation signals, and other expression control elements such as post-transcriptional regulatory elements (PREs).

The vector may comprise an origin of replication (ori) for its replication. The vector may also comprise a marker for selecting cells containing the vector. Examples of selection markers include genes conferring resistance to drugs such as neomycin, kanamycin, puromycin, hygromycin, zeocin, and ampicillin.

In one embodiment, the vector is an AAV vector. There are many serotypes of AAV, including, for example, AAV1, AAV2, AAV3, AAV4, AAV5, AAV6, AAV7, AAV8, AAV9, AAV10, AAV11, AAV12, AAV-rh10, AAV-DJ, and AAVAnc80. In one embodiment, the vector is an AAV9 vector. The AAV vector may be a natural AAV vector or a recombinant AAV vector in which, for example, a capsid protein is modified.

The AAV vector can be produced, for example, by introducing into packaging cells an AAV vector plasmid comprising a sequence encoding the constitutively active IKKβ (or a sequence encoding the constitutively active IKKβ placed downstream of a promoter) and ITRs (inverted terminal repeats) present at both ends of the sequence, together with an AAV helper plasmid comprising Rep and Cap genes required for replication and particle formation of AAV, and an adenovirus helper plasmid having adenovirus helper genes required for propagation of AAV. Examples of packaging cells include 293T cells, 293 cells, HeLa cells, COS1 cells, and COS7 cells. Viral particles released from the packaging cells can be recovered from a culture supernatant of the packaging cells by a purification method such as centrifugation, filtration, or column purification.

In the Examples described below, myocardial infarction surgery was performed on mice expressing a constitutively active IKKβ in myofibroblasts. Compared to controls, reduction of the myocardial infarction area and myocardial fibrosis was observed. In addition, myocardial infarction surgery was performed in wild-type mice, and a polynucleotide encoding a constitutively active IKKβ was administered. As a result, improvement in left ventricular ejection fraction, prolongation of survival time, suppression of myocardial fibrosis, and hyperplasia and hypertrophy of the myocardial wall were observed. Accordingly, a constitutively active IKKβ can be used for treating a cardiac disease. Without being bound by theory, expression of a constitutively active IKKβ is considered to lead to suppression of myocardial fibrosis and regeneration of myocardium.

Examples of cardiac diseases to be treated include cardiac diseases characterized by myocardial injury, such as myocardial infarction, dilated cardiomyopathy, hypertrophic cardiomyopathy, myocarditis, hypertensive cardiomyopathy, and specific cardiomyopathy, particularly myocardial infarction.

As used herein, "treating" or "treatment" refers to, in a subject suffering from a disease, alleviating or eliminating the cause of the disease, delaying or stopping progression of the disease, and/or alleviating, ameliorating, improving, or eliminating symptoms of the disease.

The subject to be treated for a cardiac disease may be any species, and is typically a mammal (e.g., human, mouse, rat, hamster, rabbit, cat, dog, cow, sheep, or monkey), particularly a human.

The administration method of the pharmaceutical composition is not particularly limited, and may be via common administration routes such as intracoronary administration, direct intramyocardial injection, oral administration, parenteral administration, injection, infusion, or instillation. The composition may be in a dosage form suitable for each administration route. In one embodiment, the pharmaceutical composition is administered intravenously. In one embodiment, the pharmaceutical composition is administered intracoronarily. In one embodiment, the pharmaceutical composition is administered by direct intramyocardial injection.

Dosage forms for oral administration include granules, fine granules, powders, coated tablets, tablets, fine powders, capsules, microcapsules, chewable preparations, liquids, suspensions, and emulsions. Dosage forms for administration by injection include general pharmaceutical dosage forms such as those for intracoronary administration, direct intramyocardial injection, intravenous injection, or drip infusion, and formulations that prolong release of an active substance. Dosage forms for intracoronary administration, direct intramyocardial injection, intravenous injection, or drip infusion include aqueous and non-aqueous injectable solutions (which may contain antioxidants, buffers, bacteriostats, tonicity agents, and the like), and aqueous and non-aqueous injectable suspensions (which may contain suspending agents, thickening agents, and the like). Injectable dosage forms may be provided in sealed ampoules or vials, or may be provided as lyophilized products to which a sterile liquid (e.g., water for injection) is added immediately before use. Injectable solutions or suspensions may be prepared from powders, granules, or tablets.

These dosage forms are produced by formulation according to conventional methods. In addition, various pharmaceutically acceptable formulation materials may be blended as needed for formulation. Formulation materials can be appropriately selected depending on the dosage form, and examples include buffering agents, surfactants, stabilizers, preservatives, excipients, diluents, additives, disintegrants, binders, coating agents, lubricants, glidants, flavoring agents, sweeteners, and solubilizers.

The dose and number of administrations of the pharmaceutical composition can be appropriately set by those skilled in the art depending on the animal species to be treated, health condition, age, body weight, administration route, dosage form, and the like, so that an effective amount of the polynucleotide encoding the constitutively active IKKβ is administered to a subject. An effective amount under a certain situation can be readily determined by routine experimentation and is within the skill and judgment of an ordinary clinician. For example, when an AAV vector comprising the polynucleotide encoding the constitutively active IKKβ is administered to a mouse, about 1×10¹¹ vg to 1×10¹² vg, about 2×10¹¹ vg to 1×10¹² vg, about 2×10¹¹ vg to 5×10¹¹ vg, about 3×10¹¹ vg to 7×10¹¹ vg, or about 4×10¹¹ vg to 6×10¹¹ vg, for example, about 2×10¹¹ vg or about 5×10¹¹ vg of the AAV vector may be administered. For example, when an AAV vector comprising the polynucleotide encoding the constitutively active IKKβ is administered to a human, about 1×10¹² vg to 1×10¹⁶ vg, about 1×10¹³ vg to 1×10¹⁵ vg, or about 1×10¹⁴ vg to 5×10¹⁴ vg, for example, about 3×10¹⁴ vg of the AAV vector may be administered. The pharmaceutical composition may be administered once, multiple times, or continuously. In the case of multiple administrations, for example, it may be administered at a frequency of once to several times per day, for example, once, twice, or three times per day, consecutively or every few days, for example, every 1, 2, 3, or 7 days. The administration period is not limited, and a drug holiday period may be provided. In one embodiment, the pharmaceutical composition is administered once. In one embodiment, the pharmaceutical composition is administered within 1 week, 6 days, 5 days, 4 days, 3 days, 2 days, or 1 day after myocardial infarction, for example, within 2 days. In one embodiment, the pharmaceutical composition is administered simultaneously with, immediately before, or immediately after revascularization after myocardial infarction.

The polynucleotide encoding the constitutively active IKKβ can be used alone, or in combination with one or more further active ingredients, particularly active ingredients for treating or preventing a cardiac disease. For example, the pharmaceutical composition may comprise one or more further active ingredients in addition to the polynucleotide encoding the constitutively active IKKβ.

Using components "in combination" means not only using a dosage form containing all of the components or using a combination of dosage forms each containing the components separately, but also administering the components simultaneously or administering any component with a delay relative to another component, as long as they are used for the treatment of a cardiac disease. Two or more further active ingredients can also be used in combination. Active ingredients suitable for combination include, for example, anti-inflammatory agents, vasodilators, thrombolytics, antiplatelet agents, anticoagulants, statins, diuretics, cardiotonics, and therapeutic agents for heart failure.

In addition to administration of the polynucleotide encoding the constitutively active IKKβ, a treatment method other than drug therapy can also be performed. Suitable treatments include, for example, coronary intervention (PCI) using a balloon catheter, a stent, or a drug-eluting stent (DES), and surgical procedures such as thrombus aspiration, gene therapy, and regenerative medicine.

An aspect provides a method of treating a cardiac disease comprising administering a polynucleotide encoding a constitutively active IKKβ to a subject in need thereof.

An aspect provides a polynucleotide encoding a constitutively active IKKβ for use in treating a cardiac disease.

An aspect provides use of a polynucleotide encoding a constitutively active IKKβ for treating a cardiac disease.

An aspect provides use of a polynucleotide encoding a constitutively active IKKβ for manufacturing a composition for treating a cardiac disease.

Another aspect provides a pharmaceutical composition for regenerating myocardium comprising a polynucleotide encoding a constitutively active IKKβ. In the present disclosure, "regenerating myocardium" or "myocardial regeneration" means proliferating cardiomyocytes in a subject in which at least a part of the myocardium is damaged, for example, a subject suffering from any of the above cardiac diseases. The pharmaceutical composition can be used in accordance with the pharmaceutical composition for treating a cardiac disease described above.

An aspect provides a method of regenerating myocardium comprising administering a polynucleotide encoding a constitutively active IKKβ to a subject in need thereof.

An aspect provides a polynucleotide encoding a constitutively active IKKβ for use in regenerating myocardium.

An aspect provides use of a polynucleotide encoding a constitutively active IKKβ for regenerating myocardium.

An aspect provides use of a polynucleotide encoding a constitutively active IKKβ for manufacturing a composition for regenerating myocardium.

Another aspect provides a pharmaceutical composition for suppressing myocardial fibrosis comprising a polynucleotide encoding a constitutively active IKKβ. In the present disclosure, "suppressing myocardial fibrosis" or "suppression of myocardial fibrosis" means preventing occurrence of myocardial fibrosis, delaying or stopping progression of myocardial fibrosis, or causing regression or reduction of myocardial fibrosis, in a subject having a risk of myocardial fibrosis or having fibrosis in a part of the myocardium, for example, a subject suffering from any of the above cardiac diseases. The pharmaceutical composition can be used in accordance with the pharmaceutical composition for treating a cardiac disease described above.

An aspect provides a method of suppressing myocardial fibrosis comprising administering a polynucleotide encoding a constitutively active IKKβ to a subject in need thereof.

An aspect provides a polynucleotide encoding a constitutively active IKKβ for use in suppressing myocardial fibrosis.

An aspect provides use of a polynucleotide encoding a constitutively active IKKβ for suppressing myocardial fibrosis.

An aspect provides use of a polynucleotide encoding a constitutively active IKKβ for manufacturing a composition for suppressing myocardial fibrosis.

For example, the disclosure provides the following embodiments.
[1] A pharmaceutical composition for treating a cardiac disease, comprising a polynucleotide encoding a constitutively active IKKβ.
[2] The pharmaceutical composition according to item 1, wherein the cardiac disease is myocardial infarction, dilated cardiomyopathy, hypertrophic cardiomyopathy, myocarditis, hypertensive cardiomyopathy, or specific cardiomyopathy.
[3] The pharmaceutical composition according to item 1 or 2, wherein the cardiac disease is myocardial infarction.
[4] A pharmaceutical composition for regenerating myocardium, comprising a polynucleotide encoding a constitutively active IKKβ.
[5] A pharmaceutical composition for suppressing myocardial fibrosis, comprising a polynucleotide encoding a constitutively active IKKβ.
[6] The pharmaceutical composition according to any one of items 1 to 5, wherein the constitutively active IKKβ comprises:
   an amino acid sequence having about 90% or more identity to the amino acid sequence of SEQ ID NO: 3, wherein the amino acids corresponding to positions 177 and 181 of SEQ ID NO: 3 are glutamic acids; or
   an amino acid sequence having about 90% or more identity to the amino acid sequence of SEQ ID NO: 4, wherein the amino acids corresponding to positions 177 and 181 of SEQ ID NO: 4 are glutamic acids.
[7] The pharmaceutical composition according to any one of items 1 to 6, wherein the constitutively active IKKβ comprises the amino acid sequence of SEQ ID NO: 3 or 4.
[8] The pharmaceutical composition according to any one of items 1 to 7, wherein the constitutively active IKKβ consists of the amino acid sequence of SEQ ID NO: 3 or 4.
[9] The pharmaceutical composition according to any one of items 1 to 8, wherein the polynucleotide encoding the constitutively active IKKβ is operably linked to a myofibroblast-specific promoter.
[10] The pharmaceutical composition according to item 9, wherein the myofibroblast-specific promoter is an SM22α promoter.
[11] The pharmaceutical composition according to item 10, wherein the SM22α promoter comprises a nucleotide sequence having about 90% or more identity to SEQ ID NO: 7 or 8.
[12] The pharmaceutical composition according to item 10 or 11, wherein the SM22α promoter comprises the nucleotide sequence of SEQ ID NO: 7 or 8.
[13] The pharmaceutical composition according to any one of items 10 to 12, wherein the SM22α promoter consists of the nucleotide sequence of SEQ ID NO: 7 or 8.
[14] The pharmaceutical composition according to any one of items 1 to 13, wherein the polynucleotide encoding the constitutively active IKKβ is contained in a vector.
[15] The pharmaceutical composition according to item 14, wherein the vector is a viral vector.
[16] The pharmaceutical composition according to item 14 or 15, wherein the vector is an adeno-associated virus (AAV) vector.
[17] The pharmaceutical composition according to item 16, wherein the AAV is AAV9.
[18] The pharmaceutical composition according to any one of items 1 to 17, which is administered within 2 days after myocardial infarction.
[19] The pharmaceutical composition according to any one of items 1 to 18, which is administered once.
[20] The pharmaceutical composition according to any one of items 1 to 19, which is administered intravenously.
[21] The pharmaceutical composition according to any one of items 1 to 19, which is administered intracoronarily.
[22] The pharmaceutical composition according to any one of items 1 to 19, which is administered by direct intramyocardial injection.
[23] The pharmaceutical composition according to any one of items 1 to 22, which is administered simultaneously with, immediately before, or immediately after revascularization after myocardial infarction.

The entire contents of the documents cited herein are incorporated herein by reference.

The embodiments described above are non-limiting and may be modified without deviating from the scope of the invention as defined by the appended claims. The following examples are non-limiting and provided only for describing the invention.

### EXAMPLES

### Production of mice expressing a constitutively active IKKβ in myofibroblasts (SM22αCre-CA (constitutive active) IKKβ mice)

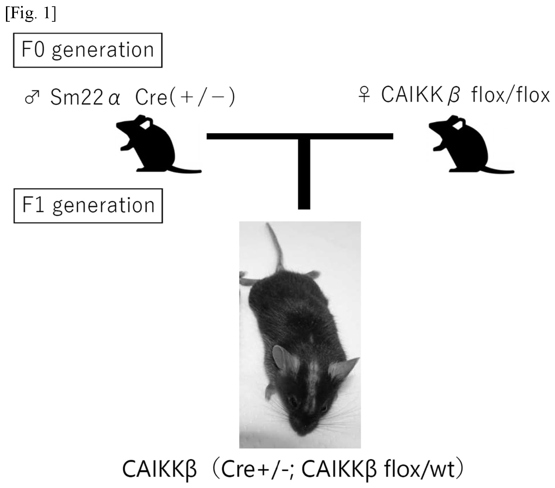

STOCK Tg(Tagln-cre)1Her/J (Jackson Lab. strain# 004746, Boucher P., et al., LRP: role in vascular wall integrity and protection from atherosclerosis. Science 300(5617):329-32) and B6.Cg-Gt(ROSA)26Sortm4(Ikbkb)Rsky/J (Jackson Lab. strain# 008242, Sasaki Y., et al., Canonical NF-kappaB activity, dispensable for B cell development, replaces BAFF-receptor signals and promotes B cell proliferation upon activation. Immunity 24(6):729-39) were crossed. From the first generation, Cre(+) mice were identified by genotyping PCR (forward primer: GCG GTC TGG CAG TAA AAA CTA TC (SEQ ID NO: 9); reverse primer: GTG AAA CAG CAT TGC TGT CAC TT (SEQ ID NO: 10)) to obtain SM22αCre-CAIKKβflox/wt mice (hereinafter referred to as CAIKKβ mice) (Fig. 1). Cre(-) mice were obtained as controls.

### Preparation of mouse myocardial infarction model

Male mice aged 8 to 12 weeks were placed under respiratory management by tracheal intubation after general anesthesia. The thoracic cavity was exposed by intercostal incision, and after incising the epicardium, the left coronary artery was identified under direct vision and ligated with a 7.0 monofilament thread (PROLENE, ETHICON). After confirming changes in myocardial color tone and myocardial motion, the chest was closed.

### Mouse echocardiography

Using Vevo2100 (Fujifilm), cardiac function after myocardial infarction was evaluated without anesthesia.

### Tissue staining

Mouse hearts were cut in the short-axis direction at the coronary artery ligation level, fixed with 4% paraformaldehyde, and embedded in paraffin or OCT. Slides were prepared from the embedded blocks at a thickness of 10 µm (paraffin block) or 6 µm (OCT block), and hematoxylin-eosin staining (HE) or Masson's trichrome staining was performed.

### Immunostaining

Mouse hearts were cut in the short-axis direction at the coronary artery ligation level, fixed with 4% paraformaldehyde, and embedded in OCT. Slides were prepared from the embedded blocks at a thickness of 6 µm (OCT block), and immunostaining was performed using ThermoFisher Cardiac Troponin Monoclonal antibody (#MA512960).

### Production of SM22α-mIKKβCA-AAV9 and SM22α-tdTomato-AAV9

Gene synthesis of promoter/enhancer sequence and transgene sequence

Genes of the mouse SM22α promoter/enhancer (546 nt) and FLAG-tagged mouse kinase-active IKKbeta (S177E/S181E, synonymous with CAIKKβ) (2,319 nt) were synthesized.

### Insertion of the synthesized genes into cis-plasmid vectors

### (1) Cis-plasmid: pAAV-SM22α-tdTOMATO-WPRE

The synthesized mouse SM22α promoter/enhancer DNA fragment was digested with restriction enzymes at the MluI and EcoRI sites, inserted into the MluI and EcoRI sites of pAAV-CMV-WPRE owned by SignaGen, thereby replacing the CMV promoter DNA sequence with the fragment to construct pAAV-SM22α-WPRE. Next, pAAV-CMV-tdTOMATO-WPRE owned by SignaGen was digested with restriction enzymes at the NheI and PmeI sites to obtain a DNA fragment containing the tdTOMATO sequence, which was inserted into the NheI and PmeI sites of the constructed pAAV-SM22α-WPRE to construct pAAV-SM22α-tdTOMATO-WPRE.

### (2) Cis-plasmid: pAAV-SM22α-mIKKβ-CA-WPRE

The pAAV-SM22α-WPRE constructed in (1) was digested with a restriction enzyme at the EcoRV site to produce blunt ends, and blunt ends of the synthesized FLAG-mouse IKK2CA (S177E-S181E) DNA fragment were inserted into the EcoRV site of pAAV-SM22α-WPRE by ligation to construct pAAV-SM22α-mIKKβ-CA-WPRE (SEQ ID NO: 11). Positions 156 to 665 of SEQ ID NO: 11 are the mouse SM22α promoter/enhancer, and positions 698 to 3010 are FLAG-mouse IKK2CA.

### Packaging into AAV and purification

AAV9 was packaged and purified as follows.
(1) SM22α-tdTOMATO-AAV9
   Cis-plasmid: pAAV-SM22α-tdTOMATO
(2) SM22α-mIKKβCA-AAV9
   Cis-plasmid: pAAV-SM22α-mIKKβ-CA

### Administration of SM22α-mIKKβCA-AAV9

Two days after myocardial infarction surgery, 2×10¹¹ vg or 5×10¹¹ vg of SM22α-mIKKβCA-AAV9 was administered via the tail vein. As controls, SM22α-tdTomato-AAV9 or no AAV was administered.

### Results

### Test 1: Myocardial infarction in CAIKKβ mice

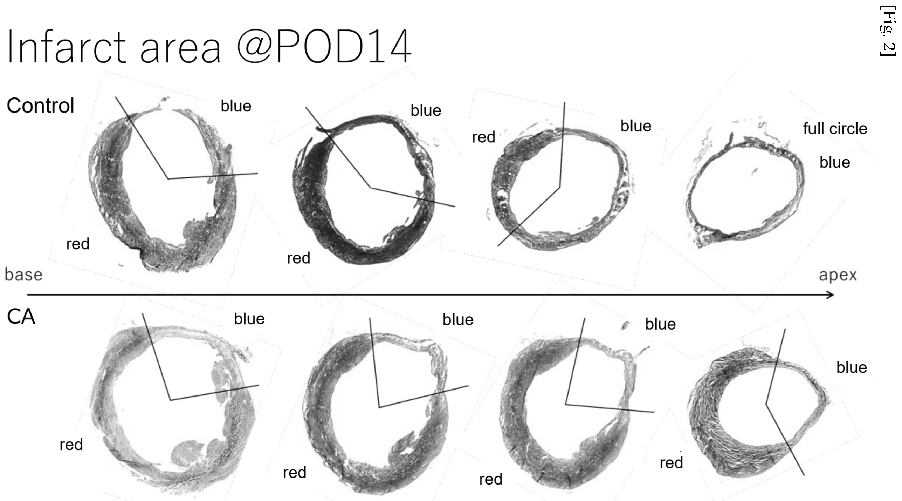

Control mice and CAIKKβ mice were subjected to myocardial infarction surgery. The mice were euthanized 14 days later and the hearts were stained with Masson's trichrome staining. Representative results are shown in Fig. 2. The region stained blue, which indicates fibrosis, was smaller in the CAIKKβ mice (lower panels) than in the control mice (upper panels). The result suggests that myocardial fibrosis was suppressed.

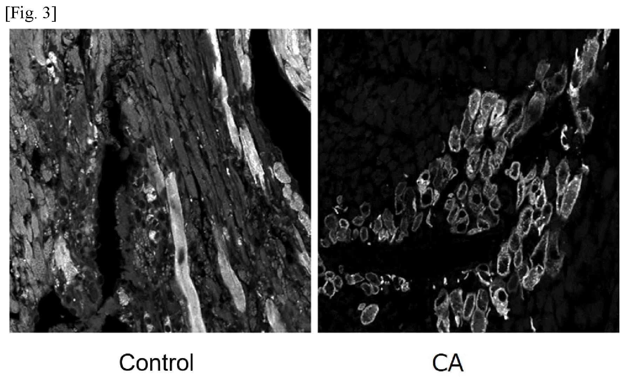

Control mice and CAIKKβ mice were subjected to myocardial infarction surgery. The mice were euthanized 14 days later and the hearts were immunostained with a troponin T antibody. Representative results are shown in Fig. 3. Cell-surface localization of troponin T was observed in cardiomyocytes of the CAIKKβ mice (right), but not in the control mice (left). Cell-surface localization of troponin T has been reported in cardiomyocytes undergoing the cell cycle, for example, in hearts of fish, in which the myocardium is regenerated, and in hearts of neonatal mice (Non-Patent Documents 1 and 2). Accordingly, the results suggest that in CAIKKβ mice cardiomyocytes proliferated and the myocardium was regenerated after myocardial infarction.

### Test 2: Effect of myofibroblast-specific CAIKKβ expression after myocardial infarction

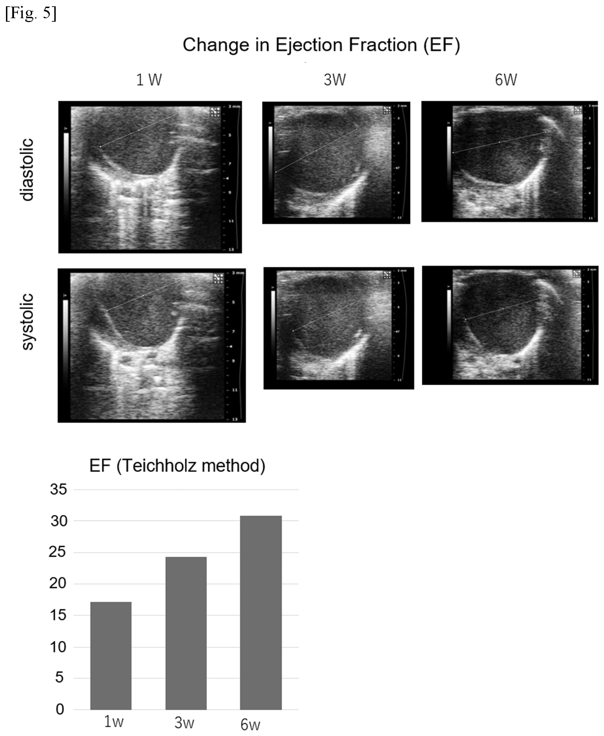

Wild-type mice (C57BL/6J, male, 8 to 12 weeks old) were subjected to myocardial infarction surgery. Two days later, SM22α-mTKKβCA-AAV9 (5×10¹¹ vg) was administered via the tail vein. The heart was observed over time by echocardiography from one week after the myocardial infarction surgery. The treatment group showed improvement in heartbeat compared with the control group. For objective evaluation, left ventricular ejection fraction (EF) in left ventricular short-axis images was calculated at the center of the infarct area by the Teichholz method. The results are shown in Fig. 5. The EF improved over time, which was consistent with the observation of the heartbeat.

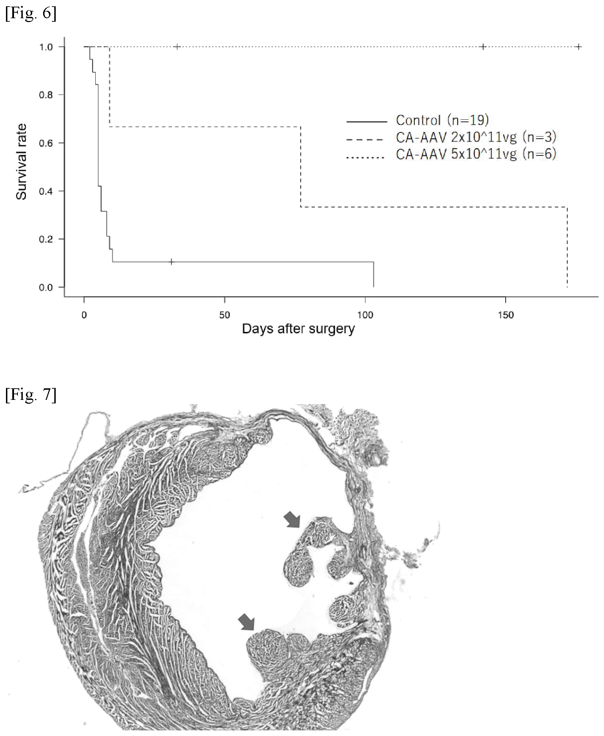

Fig. 6 shows survival rates after the myocardial infarction surgery. Survival time was longest in the mice treated with a high dose of SM22α-mIKKβCA-AAV9, followed by the mice treated with a low dose of SM22α-mIKKβCA-AAV9, and was shortest in the control mice (Logrank test: among three groups p=0.000168, administration vs no administration p=0.0000658, dose 2×10¹¹ vg vs 5×10¹¹: p=0.0499). Among 6 mice treated with a high dose of SM22α-mIKKβCA-AAV9, 2 mice did not die during the observation period and survived for 150 days or more. Observation for 4 mice was terminated 36 days after surgery (shown by censoring marks) because the start of the experiment was late. Most of the control mice died within 10 days. The treatment clearly improved the survival rate.

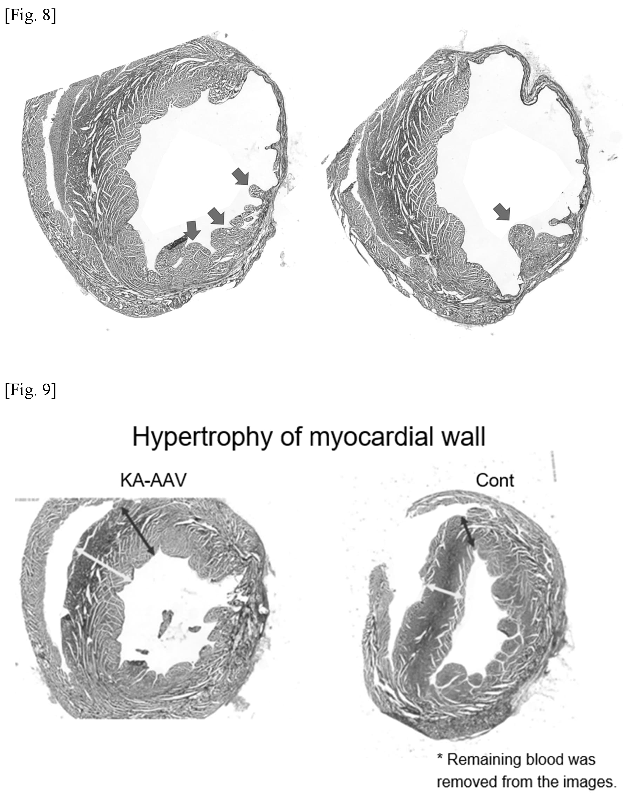

Mice were euthanized after myocardial infarction surgery, and the hearts were stained with Masson's trichrome staining. Representative results at 142 days and 176 days after the myocardial infarction surgery are shown in Figs. 7 and 8, respectively. Suppression of myocardial fibrosis and findings suggestive of myocardial proliferation (arrows) were observed in the mice treated with SM22α-mIKKβCA-AAV9 (5×10¹¹ vg). In addition, in the mice at 142 days after the myocardial infarction surgery, thickening of the myocardial wall was observed (Fig. 9: the arrows indicate the right ventricular junction and central region of the interventricular septum).

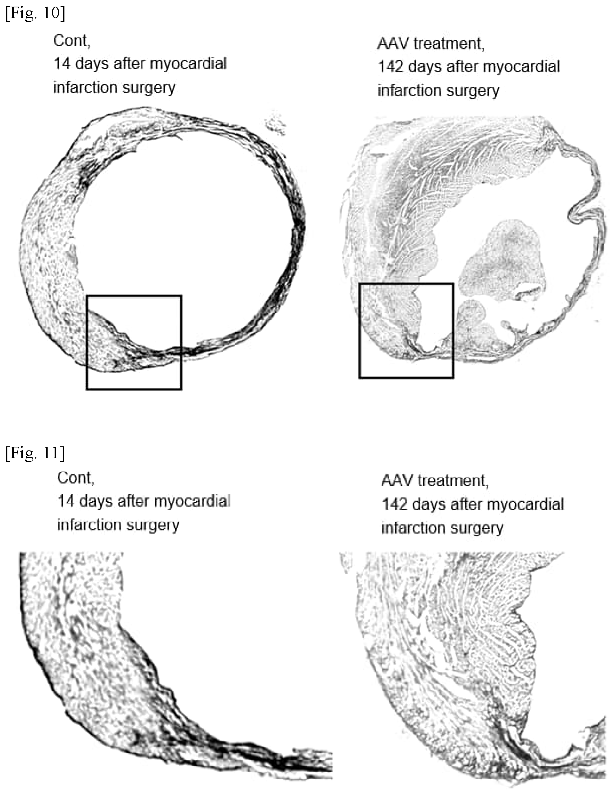

Control mice were euthanized 14 days after the myocardial infarction surgery, and mice treated with SM22α-mIKKβCA-AAV9 (5×10¹¹ vg) were euthanized 142 days after the myocardial infarction surgery, and the hearts were stained with Masson's trichrome staining. Representative results are shown in Figs. 10 and 11. The region stained blue (black in Figs. 10 and 11), which indicates fibrosis, was smaller in the mice treated with SM22α-mTKKβCA-AAV9 than in the control mice.

These results indicate that a constitutively active IKKβ suppresses myocardial fibrosis after myocardial infarction and promotes myocardial regeneration. Accordingly, a constitutively active IKKβ is effective for treating a cardiac disease characterized by myocardial injury, such as myocardial infarction.

### INDUSTRIAL APPLICABILITY

The present disclosure is applicable in the fields of medicine.

## Claims

1. A pharmaceutical composition for treating a cardiac disease, comprising a polynucleotide encoding a constitutively active IKKβ.

2. The pharmaceutical composition according to claim 1, wherein the constitutively active IKKβ comprises:
an amino acid sequence having about 90% or more identity to the amino acid sequence of SEQ ID NO: 3, wherein the amino acids corresponding to positions 177 and 181 of SEQ ID NO: 3 are glutamic acids; or
an amino acid sequence having about 90% or more identity to the amino acid sequence of SEQ ID NO: 4, wherein the amino acids corresponding to positions 177 and 181 of SEQ ID NO: 4 are glutamic acids.

3. The pharmaceutical composition according to claim 1, wherein the constitutively active IKKβ comprises the amino acid sequence of SEQ ID NO: 3 or SEQ ID NO: 4.

4. The pharmaceutical composition according to any one of claims 1 to 3, wherein the polynucleotide encoding the constitutively active IKKβ is operably linked to a myofibroblast-specific promoter.

5. The pharmaceutical composition according to claim 4, wherein the myofibroblast-specific promoter is an SM22α promoter.

6. The pharmaceutical composition according to claim 1, wherein the polynucleotide encoding the constitutively active IKKβ is contained in a vector.

7. The pharmaceutical composition according to claim 6, wherein the vector is a viral vector.

8. The pharmaceutical composition according to claim 6, wherein the vector is an adeno-associated virus (AAV) vector.

9. The pharmaceutical composition according to claim 8, wherein the AAV is AAV9.

10. The pharmaceutical composition according to claim 1, wherein the cardiac disease is myocardial infarction, dilated cardiomyopathy, hypertrophic cardiomyopathy, myocarditis, hypertensive cardiomyopathy, or specific cardiomyopathy.

11. The pharmaceutical composition according to claim 1, wherein the cardiac disease is myocardial infarction.

12. The pharmaceutical composition according to claim 11, which is administered within 2 days after myocardial infarction.

13. A pharmaceutical composition for regenerating myocardium, comprising a polynucleotide encoding a constitutively active IKKβ.

14. A pharmaceutical composition for suppressing myocardial fibrosis, comprising a polynucleotide encoding a constitutively active IKKβ.
